# EUROPÄISCHE PATENTSCHRIFT

(11) **EP 1 345 881 B1**
(45) Veröffentlichungstag und Bekanntmachung des Hinweises auf die Patenterteilung: **20.10.2004**
(21) Anmeldenummer: 01985875.2
(22) Anmeldetag: 13.12.2001
(51) Int. Cl.: C07C 51/42, C07B 63/00

(54) **VERFAHREN ZUM ABSCHRECKEN EINES HEISSEN (METH)ACRYLSAEURE ENTHALTENDEN GASGEMISCHES**
METHOD FOR RAPIDLY COOLING A HOT GAS MIXTURE CONTAINING (METH)ACRYLIC ACID
PROCEDE POUR REFROIDIR BRUSQUEMENT UN MELANGE GAZEUX TRES CHAUD CONTENANT DE L'ACIDE (METH)ACRYLIQUE

(30) Priorität: 18.12.2000 DE 10063161
(43) Veröffentlichungstag der Anmeldung: 24.09.2003
(73) Patentinhaber: BASF AKTIENGESELLSCHAFT, 67056 Ludwigshafen (DE)
(72) Erfinder: DIEHL, Volker, 67158 Ellerstadt (DE); MÜLLER-ENGEL, Klaus, Joachim, 76297 Stutensee (DE); NESTLER, Gerhard, 1070 Wien (AT)
(86) Internationale Anmeldenummer: PCT/EP2001/014634
(87) Internationale Veröffentlichungsnummer: WO 2002/050011

(56) Entgegenhaltungen:
- DE-A- 19 924 533
- GB-A- 2 053 262

## Beschreibung

Vorliegende Erfindung betrifft ein Verfahren zum raschen Abkühlen (Abschrecken) eines heißen (Meth)acrylsäure enthaltenden Gasgemisches durch direkte Kühlung mit einer Kühlflüssigkeit in einem Sprühkühler (Quench).

Unter einem Sprühkühler soll in dieser Schrift ein Apparat verstanden werden, in welchem die Kühlflüssigkeit mittels Zerstäuber in feinteilige Tröpfchen versprüht (zerteilt) und das zu kühlenden Gas durch direkten Kontakt mit der zerstäubten Kühlflüssigkeit gekühlt wird. Die versprühte Kühlflüssigkeit und das zu kühlende Gas werden dabei in der Regel im Gleichstrom durch den Sprühkühler geführt. Normalerweise wird die Kühlflüssigkeit in den auf- oder absteigenden Gasstrom versprüht.

(Meth)acrylsäure wird hier als verkürzte Schreibweise verwendet und steht für Acrylsäure oder Methacrylsäure.

(Meth)acrylsäure, entweder für sich oder in Form ihrer Ester, ist insbesondere zur Herstellung von Polymerisaten für die verschiedensten Anwendungsgebiete, z.B. Verwendung als Klebstoffe, von Bedeutung.

Unter anderem ist (Meth)acrylsäure durch katalytische Gasphasenoxidation von Alkanen, Alkanolen, Alkenen oder Alkenalen erhältlich, die 3 bzw. 4 C-Atome enthalten. Besonders vorteilhaft ist (Meth)acrylsäure z.B. durch katalytische Gasphasenoxidation von Propan, Propen, tert.-Butanol, iso-Buten, iso-Butan, iso-Butyraldehyd oder Methacrolein erhältlich. Als Ausgangsverbindungen sind aber auch als solche denkbar, aus welchen sich die eigentliche C₃-/C₄-Ausgangsverbindung während der Gasphasenoxidation erst intermediär bildet. Beispielhaft genannt sei der Methylether des tert.-Butanols.

Dabei werden diese Ausgangsgase, in der Regel mit Inertgasen wie Stickstoff, CO, CO₂, gesättigten Kohlenwasserstoffen und/oder Wasserdampf verdünnt, im Gemisch mit Sauerstoff bei erhöhten Temperaturen (üblicherweise ca. 200 bis 400°C) sowie gegebenenfalls erhöhtem Druck über übergangsmetallische (z.B. Mo, V, W und/oder Fe enthaltende) Mischoxidkatalysatoren geleitet und oxidativ in die (Meth)acrylsäure umgewandelt (vgl. z.B. DE-A 4 405 059,

EP-A 253 409, EP-A 92 097, DE-A 4 431 957, DE-A 4 431 949, CN-A 1 105 352, WO 97/36849 und EP-A 608 838).

Aufgrund zahlreicher im Verlauf der katalytischen Gasphasenoxidation erfolgender Parallel- und Folgereaktionen sowie aufgrund der mitzuverwendenden inerten Verdünnungsgase wird bei der katalytischen Gasphasenoxidation jedoch keine reine (Meth)acrylsäure sondern ein heißes Reaktionsgasgemisch erhalten, das im wesentlichen (Meth)acrylsäure, die inerten Verdünnungsgase und Nebenprodukte enthält, aus welchem die (Meth)acrylsäure abgetrennt werden muß. Neben von (Meth)acrylsäure vergleichsweise einfach zu entfernenden und bei Folgeverwendungen der (Meth)acrylsäure weniger störenden Nebenprodukten wie z.B. Essigsäure, enthält das heiße Reaktionsgasgemisch häufig mit (Meth)acrylsäure eng verwandte und daher von (Meth)acrylsäure schwer abtrennbare niedere Aldehyde wie Formaldehyd, Acetaldehyd, Acrolein, Methacrolein, Propionaldehyhd, n-Butyraldehyd, Propionsäure, Benzaldehyd, Furfural und Crotonaldehyd sowie zusätzlich gegebenenfalls Maleinsäureanhydrid (bezogen auf die im Reaktionsgasgemisch enthaltene Menge an (Meth)acrylsäure beträgt die Gesamtmenge dieser, bei Folgeverwendungen häufig erheblich störenden, Nebenkomponenten in der Regel ≤2 Gew.-%, meist ≥ 0,05 Gew.-%).

Aus der DE-A 19 740 252, der DE-A 19 740 253, der DE-A 19 833 049, der DE-A 19 814 375, der DE-A 19 814 421, der DE-A 19 814 449, der DE-A 10 053 086, der DE-A 10 039 025, der DE-A 19 924 533 und der DE-A 19 924 532 ist bekannt, daß aus (Meth)acrylsäure enthaltenden heißen Produktgasgemischen von heterogen katalysierten Gasphasen-Partialoxidationen von C₃/C₄-Vorläufern der (Meth)acrylsäure eine Grundabtrennung der im heißen Produktgasgemisch enthaltenen (Meth)acrylsäure dadurch möglich ist, daß man das heiße Produktgasgemisch nach direkter Vorkühlung (Abschrecken, Quenchen) mit einer Kühlflüssigkeit partiell oder vollständig kondensiert oder in ein geeignetes Absorptionsmittel (z.B. Wasser, (Meth)acrylsäure, oligomere (Meth)acrylsäure (Michael-Addukte), hochsiedende organische Flüssigkeiten und Mischungen aus den vorgenannten Flüssigkeiten) aufnimmt.

Beispielsweise durch Entfernung des Absorptionsmittels (und gegebenenfalls zuvor erfolgte Desorption von eine geringe Absorptionsmittellöslichkeit aufweisende Verunreinigungen durch Abstreifen, z.B. mit Luft) über extraktive, destillative und/oder kristallisative Trennverfahren (z.B. Entfernung des Absorptionsmittels Wasser durch Destillation, azeotrope Destillation oder extraktive Abtrennung der Säure aus der wäßrigen Lösung und anschließende destillative Entfernung des Extraktionsmittels) und/oder nach Anwendungen von sonstigen Trennschritten wird häufig eine (Meth)acrylsäure erhalten, die als Roh-(Meth)acrylsäure bezeichnet wird (vgl. z.B. EP-A 297 445, DE-PS 2 136 306).

Zur Durchführung der direkten Kühlung des heißen Produktgasgemisches der heterogen katalysierten Gasphasen-Partialoxidation von C₃-/C₄-Vorläufern der (Meth)acrylsäure mit einer Kühlflüssigkeit empfiehlt die DE-A 19 924 533 die Verwendung von an Einbauten freien Sprühkühlern.

Zur Zerstäubung der Kühlflüssigkeit sieht die DE-A 19 924 533 die Verwendung von Zerstäuberdüsen vor. Derartigen Düsen kann die Kühlflüssigkeit z.B. unter Druck zugeführt werden. Die Zerteilung der Kühlflüssigkeit erfolgt kann dadurch, daß sie nach Erreichen einer bestimmten Mindestgeschwindigkeit in der Düsenbohrung entspannt wird. Ferner werden für den vorgenannten Zweck Einstoffdüsen wie z.B. Drallkammerdüsen (Hohl- oder Vollkegeldüsen) angeboten (z.B. von der Fa. Düsen-Schlick GmbH, DE, oder von der Sprayring Systems Deutschland GmbH).

Im einfachen Fall kann die verwendete Kühlflüssigkeit mit der für die nachfolgende Absorption verwendeten Flüssigkeit chemisch identisch sein.

Nachteilig an den im Stand der Technik zur Zerstäubung verwendeten Zerstäuberdüsen ist jedoch, daß sie sehr leicht verstopfen. Dies ist u.a. darauf zurückzuführen, daß das zu kühlende Reaktionsgasgemisch mit (Meth)acrylsäure einen Bestandteil enthält, der eine hohe Neigung zur radikalischen Polymerisation aufweist. Die resultierenden Polymerisate sind in der Regel klebrig und führen leicht zur Verstopfung der Sprühdüsen.

Eine nachträgliche Reinigung der verstopften Teilen, z.B. durch Auskochen mit wäßriger Natronlauge, ist aufwendig und umweltbelastend.

Aufgabe der vorliegenden Erfindung war es daher, ein verbessertes Verfahren zum raschen Abkühlen eines heißen (Meth)acrylsäure enthaltenden Gasgemisches durch direkte Kühlung mit einer Kühlflüssigkeit in einem Sprühkühler zur Verfügung zu stellen.

Demgemäß wurde ein Verfahren zum Abkühlen eines heißen (Meth)acrylsäure enthaltenden Gasgemisches durch direkte Kühlung mit einer Kühlflüssigkeit in einem Sprühkühler gefunden, das dadurch gekennzeichnet ist, daß zum Zerstäuben der Kühlflüssigkeit im Sprühkühler wenigstens ein Prallzerstäuber verwendet wird.

Häufig bewirkt der Prallzerstäuber erfindungsgemäß eine Tröpfchengröße von 0,1 mm bis 5 mm.

Bei Prallzerstäubern wird die Zerstäubung dadurch bewirkt, daß wenigstens ein Strom der Kühlflüssigkeit (Quenchflüssigkeit) entweder auf wenigstens einen zweiten Strom der Kühlflüssigkeit und/oder auf eine Prallplatte trifft.

Erfindungsgemäß bevorzugt sind Prallzerstäuber, bei denen die Zerstäubung dadurch bewirkt wird, daß wenigstens ein Strom der Kühlflüssigkeit auf eine Prallplatte (z.B. aus Stahl) trifft (Prallplattenzerstäuber).

Erfindungsgemäß günstig ist es, wenn der auf die Prallplatte geführte Strom der Kühlflüssigkeit eine Strömungsgeschwindigkeit von 20 bis 80 km/h aufweist.

Die Führung der Quenchflüssigkeit erfolgt in zweckmäßiger Weise in einfachen Rohren (z.B. aus Stahl), die gegen das Ende hin vorzugsweise verjüngt sind.

Der Abstand zwischen der Austrittsöffnung des Rohres und der Prallplatte beträgt erfindungsgemäß häufig 5 bis 30 cm, mit Vorteil 10 bis 20 cm. Die Größe und Form der Prallplatte kann in weiten Grenzen variieren. In der.Regel ist die Prallplatte rund und ihr Durchmesser beträgt häufig das 1-bis 20-fache, mit Vorteil das 1- bis 5-fache des Durchmessers der Austrittsöffnung des Rohres.

Im Normalfall ist die Prallplatte eben. Die Prallplatte kann aber auch konkav oder konvex geformt sein.

Erfindungsgemäß von Vorteil ist es ferner, wenn die Oberfläche der verwendeten Prallplatte mit Polymerisationsinhibitoren (die die radikalische Polymerisation von (Meth)acrylsäure zu inhibieren vermögen) ausgerüstet ist, wie es z.B. die DE-A 19 915 116, die DE-A 19 915 104 und die DE-A 10 055 645 sowie die in diesen Schriften zitierten Referenzen empfehlen. Zweckmäßigerweise wird die Polymerisationsinhibierung der Prallplatte so gewählt, daß sie in der Quenchflüssigkeit wenig löslich ist.

Erfindungsgemäß günstig ist es ferner, wenn die verwendete Quenchflüssigkeit einen Polymerisationsinhibitor zugesetzt enthält. Zweckmäßigerweise wird der zuzusetzende Polymerisationsinhibitor so gewählt, daß er in der einzusetzenden Menge in der Quenchflüssigkeit löslich ist.

Als vorgenannte Polymerisationsinhibitoren geeignet sind z.B. phenolische Verbindungen, Amine, Nitroverbindungen, phosphor- oder schwefelhaltige Verbindungen, Hydroxylamine, N-Oxide und Chinone. Beispielsweise kommen alle Polymerisationsinhibitoren in Betracht, die in der DE-A 10 053 086 aufgeführt sind.

In typischer Weise weist das erfindungsgemäß zu kühlende heiße (Meth)acrylsäure enthaltende Gasgemisch eine Temperatur von 200 bis 400°C auf und wird durch die direkte Kühlung üblicherweise auf eine Temperatur von 100 bis 180°C abgekühlt. Die Temperatur der erfindungsgemäß eingesetzten Kühlflüssigkeit weist zu diesem Zweck normalerweise eine Temperatur von 70 bis 170°C auf.

Als erfindungsgemäß zu verwendende Quenchflüssigkeiten kommen z.B. hochsiedende inerte hydrophobe organische Flüssigkeiten in Betracht, wie sie in der DE-A 2 136 396 und in der DE-A 4 308 087 aufgeführt sind. Dies sind im wesentlichen Flüssigkeiten, deren Siedepunkt bei Normaldruck oberhalb von 160°C liegt. Beispielhaft genannt seien Mittelölfraktionen aus der Paraffindestillation, Diphenylether, Diphenyl, oder Mischungen der vorgenannten Flüssigkeiten wie z.B. ein Gemisch aus 70 bis 75 Gew.-% Diphenylether und 25 bis 30 Gew.-% Diphenyl. Günstig ist z.B. die Verwendung eines Gemisches bestehend aus einer Mischung aus 70 bis 75 Gew.-% Diphenylether und 25 bis 30 Gew.-% Diphenyl, sowie, bezogen auf diese Mischung, 0,1 bis 25 Gew.-% o-Dimethylphthalat. Geeignet sind als Quenchflüssigkeiten aber auch Alkylester von Carbonsäuren deren Siedepunkt bei Normaldruck (1 atm) wenigstens 160°C beträgt und deren Schmelzpunkt ≤ 30°C beträgt (vgl. z.B. DE-A 2 241 714).

Selbstverständlich kann erfindungsgemäß als Quenchflüssigkeit aber auch Wasser, (Meth)acrylsäure oder ein Gemisch aus oligomerer (Meth)acrylsäure (Michael-Addukte) eingesetzt werden (vgl. z.B. DE-A 19 814 387).

In zweckmäßiger Weise handelt es sich bei dem erfindungsgemäß zu verwendenden Sprühkühler um ein weites senkrecht stehendes Rohr oder um einen, bevorzugt zylinderförmigen, Schacht, in welche man z.B. das abzuschreckende, (Meth)acrylsäure enthaltende heiße Reaktionsgasgemisch in vorteilhafter Weise von oben (bevorzugt in der Mitte) einströmen läßt.

Im oberen Teil des Sprühkühlers befinden sich, vorzugsweise symmetrisch angeordnet und gleichmäßig über den Querschnitt verteilt, drei bis sechs Zuführrohre (die zweckmäßigerweise über eine Ringleitung mit Quenchflüssigkeit versort werden), über die die Quenchflüssigkeit zugeführt wird. Die Zuführrohre führen die Quenchflüssigkeit auf ebenfalls gleichmäßig verteilte und symmetrisch angeordnete Prallplatten. Neben den Zuführrohren und den Prallplatten enthält der Sprühkühler in der Regel keine weiteren Einbauten.

Relativ zum Einströmquerschnitt des heißen Reaktionsgasgemisches können die Prallplatten sowohl vertikal (parallel zu Richtung der Gasströmung) als auch schräg angeordnet sein. Dadurch wird ein Besprühen des Quenchkopfes mit Quenchflüssigkeit sichergestellt. Eine horizontale Anordnung ist in der Regel weniger zweckmäßig.

Die in das heiße Reaktionsgasgemisch zersprühte Quenchflüssigkeit und das heiße Reaktionsgasgemisch bewegen sich im Gleichstrom von oben nach unten durch den Sprühkühler. Am unteren Ende des Sprühkühlers wird die Quenchflüssigkeit gesammelt, abgeleitet und nach Kühlung zum Quenchen wiederverwendet. Das gekühlte Reaktionsgasgemisch verläßt den Sprühkühler in der Regel über einen am entgegengesetzten Ende angebrachten Auslaß und kann z.B. zur weiteren Aufarbeitung einem Absorber zugeführt werden.

Derartige Reaktionsgasgemische können beispielsweise wie folgt zusammengesetzt sein:
1 bis 30 Gew.-% Acrylsäure,
0,01 bis 3 Gew.-% Essigsäure,
0,01 bis 1 Gew.-% Propionsäure,
0,01 bis 0,5 Gew.-% Maleinsäure/Maleinsäureanhydrid,
0,05 bis 1 Gew.-% Acrolein,
0,05 bis 1 Gew.-% Formaldehyd,
0,01 bis 1 Gew.-% Furfural,
0,01 bis 0,5 Gew.-% Benzaldehyd,
0,01 bis 1 Gew.-% Propen,
0,05 bis 10 Gew..% Sauerstoff,
1 bis 30 Gew.-% Wasser und
als Restmenge inerte Gase wie z.B. Stickstoff, Kohlendioxide, Methan und Propan.

Die Gasphasenoxidationen des Propens selbst kann z.B. in zwei aufeinanderfolgenden Oxidationsstufen durchgeführt werden, wie sie in der EP-A 700 714 und in der EP-A 700 893 beschrieben sind. Selbstverständlich können aber auch die in der DE-A 19 740 253 sowie die in der DE-A 19 740 252 zitierten Gasphasenoxidationen zur Anwendung kommen.

Im Rahmen des erfindungsgemäßen Verfahrens besteht auch die Möglichkeit die Prallplatten indirekt zu kühlen.

### Beispiele

### 1. Vergleichsbeispiel

150 000 Nm³/h eines durch katalytische Gasphasenoxidation von Acrolein wie in Beispiel B1 der DE-A 4 302 991 gewonnenen, Acrylsäure enthaltenden, Reaktionsgasgemisches wurden mit einer Temperatur von 270°C von oben mittig einem zylinderförmigen (Innendurchmesser = 3 m) Sprühkühler zugeführt.

Als Kühlflüssigkeit wurde ein Gemisch aus 57,4 Gew.-% Diphenylether, 20,7 Gew.-% Diphenyl und 21,9 Gew.-% o-Dimethylphthalat mit einer Temperatur von 150°C verwendet. Die Kühlflüssigkeit war mit 3000 gew.ppm Phenothiazin polymerisationsinhibiert.

Die Kühlflüssigkeit wurde über sechs über den Querschnitt des Sprühkühlers gleichmäßig verteilte, nach unten (d.h. in Strömungsrichtung des Reaktionsgasgemisches) gerichtete Vollkegelsprühdüsen (ihre Entfernung zur Wandung des Sprühkühlers betrug 75 cm) in den Sprühkühler eingesprüht.

Nach einer Betriebsdauer von sieben Tagen stellten sich Betriebsstörungen ein. Detaillierte Analysen führten zu dem Ergebnis, daß die Betriebsstörungen auf eine verminderte Sprühfähigkeit der Sprühdüsen zurückzuführen war. Genauere Untersuchungen ergaben, daß die Minderung durch Ablagerungen von Polyacrylsäure in den Düsen verursacht wurde.

### 2. Ausführungsbeispiel

Es wurde wie im Vergleichsbeispiel verfahren. Die sechs Sprühdüsen wurden jedoch durch drei Prallplattenzerstäuber (die über den Querschnitt des Sprühkühlers ebenfalls gleichmäßig verteilt waren) ersetzt. Die Prallplatten waren kreisförmig, relativ zum Einströmquerschnitt vertikal angeordnet, und wiesen einen Durchmesser von 25 cm auf. Der Abstand der Prallplatten zur Wandung des Sprühkühlers betrug 50 cm. Die Kühlflüssigkeit wurde über Rohre mit einer Geschwindigkeit von 50 km/h auf die Prallplatten geführt. Der Abstand von der Rohrausflußöffnung bis zur Prallplatte lag bei 15 cm. Die erzielte Kühlwirkung entsprach der im Vergleichsbeispiel erzielten Kühlwirkung. Auch nach 30 Tagen ununterbrochenen Betriebs stellten sich keine Betriebsstörungen ein.

## Patentansprüche

1. Verfahren zum Abkühlen eines heißen (Meth)acrylsäure enthaltenden Gasgemisches durch direkte Kühlung mit einer Kühlflüssigkeit in einem Sprühkühler, **dadurch gekennzeichnet, daß** zum Zerstäuben der Kühlflüssigkeit im Sprühkühler wenigstens ein Prallzerstäuber verwendet wird.

2. Verfahren nach Anspruch 1, **dadurch gekennzeichnet, daß** der Prallzerstäuber ein Prallplattenzerstäuber ist.

3. Verfahren nach Anspruch 2, **dadurch gekennzeichnet, daß** die Prallplatte des Prallplattenzerstäubers mit Polymerisationsinhibitoren ausgerüstet ist.

4. Verfahren nach einem der Ansprüche 1 bis 3, **dadurch gekennzeichnet, daß** als Kühlflüssigkeit eine organische Flüssigkeit, Wasser, (Meth)acrylsäure, (Meth)acrylsäureoligomere und/oder eine Mischung der genannten Flüssigkeiten verwendet wird.

5. Verfahren nach einem der Ansprüche 1 bis 4, **dadurch gekennzeichnet, daß** das heiße Gasgemisch das Produktgasgemisch einer heterogen katalysierten Gasphasenoxidation von C₃-/C₄-Vorläufern der (Meth)acrylsäure ist.

## Claims

1. A process for cooling a hot gas mixture comprising (meth)acrylic acid by direct cooling by means of a cooling liquid in a spray cooler, wherein at least one impingement atomizer is used for atomizing the cooling liquid in the spray cooler.

2. A process as claimed in claim 1, wherein the impingement atomizer is an impingement plate atomizer.

3. A process as claimed in claim 2, wherein the impingement plate of the impingement plate atomizer is provided with polymerization inhibitors.

4. A process as claimed in any of claims 1 to 3, wherein the cooling liquid used is an organic liquid, water, (meth)acrylic acid, (meth)acrylic acid oligomers and/or a mixture of these liquids.

5. A process as claimed in any of claims 1 to 4, wherein the hot gas mixture is the product gas mixture from a heterogeneously catalyzed gas-phase oxidation of C₃/C₄ precursors of (meth)acrylic acid.

## Revendications

1. Procédé pour le refroidissement d'un mélange gazeux chaud contenant de l'acide (méth)acrylique par refroidissement direct au moyen d'un liquide de refroidissement dans un refroidisseur à pulvérisation, **caractérisé en ce que** l'on utilise, pour la pulvérisation du liquide de refroidissement dans le refroidisseur à pulvérisation, au moins un pulvérisateur à impact.

2. Procédé selon la revendication 1, **caractérisé en ce que** le pulvérisateur à impact est un pulvérisateur à impacteur.

3. Procédé selon la revendication 2, **caractérisé en ce que** l'impacteur du pulvérisateur à impact est muni d'inhibiteurs de polymérisation.

4. Procédé selon l'une quelconque des revendications 1 à 3,**caractérisé en ce que** l'on utilise comme liquide de refroidissement un liquide organique, de l'eau, de l'acide (méth)acrylique, des oligomères d'acide (méth)acrylique et/ou un mélange des liquides mentionnés.

5. Procédé selon l'une quelconque des revendications 1 à 4, **caractérisé en ce que** le mélange gazeux chaud est le mélange gazeux de produits d'une oxydation en phase gazeuse à catalyse hétérogène de précurseurs en C₃ / C₄ de l'acide (méth)acrylique.
